# EUROPÄISCHE PATENTSCHRIFT

(11) **EP 1 290 981 B1**
(45) Veröffentlichungstag und Bekanntmachung des Hinweises auf die Patenterteilung: **09.11.2011**
(21) Anmeldenummer: 02018800.9
(22) Anmeldetag: 22.08.2002
(51) Int. Cl.: A61B 18/24, A61N 5/06, A61B 5/042

(54) **Herzkatheter mit optimierter Sonde**
Heart catheter with optimized sensor
Catheter cardiaque avec senseur optimisé

(30) Priorität: 24.08.2001 DE 10141487
(43) Veröffentlichungstag der Anmeldung: 12.03.2003
(73) Patentinhaber: LasCor GmbH Laser-Medizintechnik, 81379 München (DE)
(72) Erfinder: Weber, Dietmar, 82067 Zell (DE)
(74) Vertreter: Keilitz, Wolfgang

(56) Entgegenhaltungen:
- WO-A-01/39682
- US-A- 4 862 887
- US-A- 5 109 859
- US-A- 6 143 019
- HELMUT P. WEBER ET AL.: "Laser Catheter Coagulation of Normal and Scarred Ventricular Myocardium in Dogs", LASERS IN SURGERY AND MEDICINE, vol. 22, 1998, pages 109-119,

## Beschreibung

Die Erfindung betrifft einen Herzkatheter, insbesondere zur Behandlung subendokardialer Arrhythmie, mit einem schlauchförmigen Führungskatheter, in dem eine Sonde zum Lokalisieren pathologischer Areale bewegbar und senkrecht auf die Herzwand aufsetzbar ist.

Derartige Herzkatheter werden dazu verwendet, die Reizleitung in pathologischen Bereichen einer Herzwand abzuschwächen oder zu unterbrechen. Dabei nutzt der Herzkatheter das Prinzip der kontaktlosen Laserbestrahlung zur Erzeugung tiefreichender Koagulationsnekrosen im Myokard (Herzwand). Die Laserbestrahlung hat insbesondere den Vorteil hat, dass keine Überhitzungen, Verkohlungen oder Kraterbildungen, wie z.B. bei einem HF-Katheter (HF: Hochfrequenz), im behandelten Gewebe auftreten.

Der gattungsgemäße Herzkatheter hat eine Sonde, die senkrecht auf das zu behandelnde Gewebe aufgesetzt und mittels der eine optische Faser in Abstand zur Gewebeoberfläche gehalten wird. Aus der DE3718139C1 ist das grundlegende Prinzip eines Herzkatheters mit einer Sonde für senkrechte Laserapplikation bekannt. Die dargestellte Sonde hat mehrere Fühler (Elektroden), die in der Herzwand verankert werden und zur Ableitung elektrischer Potentiale dienen. Bei dieser Gestaltung der Elektroden besteht jedoch die Gefahr, die Herzinnenhaut zu durchstechen oder aufzuschlitzen.

Ein anderer Herzkatheter mit senkrecht aufgesetzter Sonde ist in der US-Patentschrift 5,500,012 beschrieben. Diese Sonde hat zwei stabförmige, nach vorne ragende Elektroden, die in die Herzinnenwand eindringen, bis die distale Spitze einer optischen Faser an der Herzinnenwand anliegt. Nachteilig ist dabei die kleine Kontaktfläche der Elektroden, der geringe Abstand der Elektroden zueinander und die dadurch bedingte schlechte Auflösung der elektrischen Potentiale. Wegen des unmittelbaren Kontakts der Faserspitze mit dem Gewebe und der fehlenden Kühlung werden in der Regel Löcher in der Herzwand erzeugt.

Aus der DE 4205336 C1 ist ein Herzkatheter mit einer Sonde bekannt, die den im Herzen vorhandenen Blutstrom mittels einer zeltartigen Abschirmung weitgehend vom Bestrahlungsraum fernhält. Der Herzkatheter umfasst einen schlauchförmigen Führungskatheter und einen flexiblen Führungsschlauch zum Bewegen einer Sonde innerhalb des Führungskatheters. Der Sondenkörper umfasst außerdem keine Aufnahme für eine optische Faser, in der die optische Faser in einem.definierten Abstand zum Gewebe befestigt werden kann. Zwischen den Elektroden der Sonde ist eine Folie gespannt, die einen relativ geschützten Bestrahlungsraum definiert. Diese Anordnung ist jedoch relativ instabil und nicht geeignet, den im Herzen vorherrschenden Druckverhältnissen ausreichend standzuhalten. Außerdem ist die Herstellung dieser Sonde relativ kompliziert.

Die WO-A-01/39 682 offenbart einen Herzkatheter mit einer Sonde und einer optischen Faser, wobei der Sondenkörper einen distalen Abschnitt mit einem Hohlraum aufweist, in dem sich das von der optischen Faser ausgestrahlte Licht bis auf das zu behandelnde Gewebe ausbreiten kann. Dieser Katheter ist jedoch nicht geeignet, ihn durch enge und stark gekrümmte Blutgefäße hindurch zu bewegen.

Die US-A-6,143,019 offenbart ebenfalls einen Herzkatheter mit einer optischen Faser.

Die optische Faser der US-A-6,143,019 ist relativ zum katheter schlauch bewegbar.

Es ist daher die Aufgabe der folgenden Erfindung, einen Herzkatheter mit einer Sonde zu schaffen, die besonders einfach herstellbar und wesentlich stabiler ist. Gleichzeitig soll die Herzwand bei der Anwendung nicht verletzt werden.

Gelöst wird diese Aufgabe durch die im Patentanspruch 1 angegebenen Merkmale. Weitere Ausführungsformen der Erfindung sind Gegenstand von Unteransprüchen.

Der erfindungsgemäße Herzkatheter umfaßt einen schlauchförmigen Führungskatheter und eine Sonde zum Lokalisieren der zu behandelnden Areale, die senkrecht auf die zu behandelnde Fläche aufgesetzt wird, wobei die Sonde einen Sondenkörper mit einer Aufnahme für eine optische Faser aufweist. Ferner umfaßt der erfindungsgemäße Herzkatheter eine Betätigungseinrichtung zum Vor- und Zurückschieben der Sonde in Axialrichtung des Führungskatheters, sowie eine optische Faser, die im Sondenkörper gelagert ist. Der wesentliche Gedanke der Erfindung besteht darin, den distalen Abschnitt des Sondenkörpers mit einem zum Gewebe hin offenen bzw. lichtdurchlässigen Hohlraum auszubilden, der umfangsseitig von einer im wesentlichen nicht-flexiblen Wand umgeben ist und der vorzugsweise einen größeren Querschnitt aufweist als die Aufnahme für die optische Faser. Dadurch ist es möglich, die Sonde stabil auf das Endokard (die Herzinnenhaut) aufzusetzen und in einer definierten Position zu halten. Die den Hohlraum umgebende Wand des distalen Abschnitts ist dabei so stabil gebildet, dass sie dem äußeren Blutdruck und dem Druck des schlagenden Herzen ohne weiteres standhält und in der Lage ist, das Blut aus dem Raum um das distale Faserende fernzuhalten, damit sich das ausgestrahlte Licht ungehindert im Hohlraum ausbreiten kann. Dadurch kann verhindert werden, dass der distale Sondenrand in den Strahlengang gedrückt wird, wodurch Verschmorungen entstehen würden.

Zum Lokalisieren der zu behandelnden Areale umfasst die Sonde vorzugsweise eine oder mehrere Elektroden. Diese könnten aber auch am äußeren Führungskatheter angebracht sein.

Der Sondenkörper ist vorzugsweise einstückig gebildet und umfasst einen proximalen und den distalen Abschnitt. Der proximale Abschnitt dient vorzugsweise zur Verbindung mit der Betätigungseinrichtung, der distale Abschnitt ist so dimensioniert, dass sich das von der Faser ausgestrahlte Licht in dem Hohlraum, der gleichzeitig einen Behandlungsraum bildet, kegelförmig auf das Gewebe ausbreiten kann, wenn die Faserspitze in Abstand zum Gewebe angeordnet ist.

Um bei einer Bestrahlung des Herzens eine Überhitzung der Herzinnenhaut zu vermeiden oder auch um Blut aus dem Raum um das distale Faserende fernzuhalten, wird vorzugsweise eine physiologische Lösung, insbesondere physiologische Kochsalzlösung, in den Hohlraum eingespült.

Der Sondenkörper und insbesondere das distale Ende des Sondenkörpers kann Öffnungen, wie z.B. Kerben aufweisen, durch die die Spülflüssigkeit austreten kann.

Gemäß einer bevorzugten Ausführungsform der Erfindung umfaßt der Sondenkörper mehrere, insbesondere drei, Elektroden, die vorzugsweise am Außenumfang des distalen Abschnitts befestigt sind. Durch den großen Abstand der Elektroden voneinander wird die Empfindlichkeit der Sonde verbessert und eine hohe Aussagekraft der von den Elektroden abgeleiteten EKG-Kurven erreicht.

Außerdem sind die Elektroden vorzugsweise nicht flexibel gebildet und ermöglichen daher eine stabile Positionierung der Sonde auf dem Endokard.

Gemäß einer bevorzugten Ausgestaltungsform der Erfindung ragen die Elektroden nicht oder nur geringfügig (vorzugsweise weniger als 0,5mm, insbesondere 0,2 oder 0,1mm) über das distale Ende des Sondenkörpers hervor. Eine Verletzung des Endokarts durch die Elektroden ist somit nahezu ausgeschlossen.

Die Elektroden sind vorzugsweise plättchenförmig gebildet und liegen vorzugsweise an der Außenseite des distalen Abschnitts des Sondenkörpers. Gegenüber stabförmigen Elektroden ist dadurch ein relativ breitflächiger Kontakt mit der Herzwand und somit eine stark verringerte Verletzungsgefahr gegeben.

Durch die flach anliegende Positionierung der Elektroden, die zudem in Ausnehmungen versenkt angebracht und bündig mit der Außenwand des distalen Abschnitts abschließen können, ist darüber hinaus ein leichtes Durchschieben der Sonde durch Einführschleusen, Führungskatheter oder hemostatische Ventile möglich.

Gemäß einer bevorzugten Ausgestaltung der Erfindung können die distalen Enden der Elektroden geriffelt bzw. gewellt sein, wodurch eine noch bessere Verankerung der Sonde erzielt werden kann.

Der Sondenkörper hat einen oder mehrere Durchlässe zum Zuführen von Spülflüssigkeit in den Hohlraum.

Der Sondenkörper selbst besteht vorzugsweise aus einem nichtelastischen Material, wie z.B. Kunststoff und ist vorzugsweise einstückig gebildet.

Gemäß der Erfindung ist die Betätigungseinrichtung ein flexibler Schlauch, der am proximalen Abschnitt des Sondenkörpers befestigt ist. Der proximale Abschnitt des Sondenkörpers ist so dimensioniert, dass er in den Schlauch eingeführt und mit diesem verbunden, insbesondere verklebt werden kann. Der Schlauch dient ferner zum Zuleiten von Spülflüssigkeit in den Hohlraum.

Im befestigten Zustand schließt der Schlauch an seinem Außenumfang vorzugsweise bündig mit der Sonde ab.

Die Erfindung wird nachstehend anhand der beigefügten Zeichnungen beispielhaft näher erläutert. Es zeigen:
Figur 1 eine Schnittansicht eines Herzkatheters mit einem Führungskatheter und einer darin bewegbaren Sonde;
Figur 2 eine Schnittansicht der Sonde von Figur 1 gemäß einem Ausführungsbeispiel der Erfindung;
Figur 3 eine seitliche Außenansicht der Sonde von Figur 2;
Figur 4 eine Aufsicht auf die Sonde von Figur 2;
Figur 5 eine Seitenansicht einer plättchenförmigen Elektrode; und
Fig. 6 eine Aufsicht auf die Elektrode von Fig. 5.

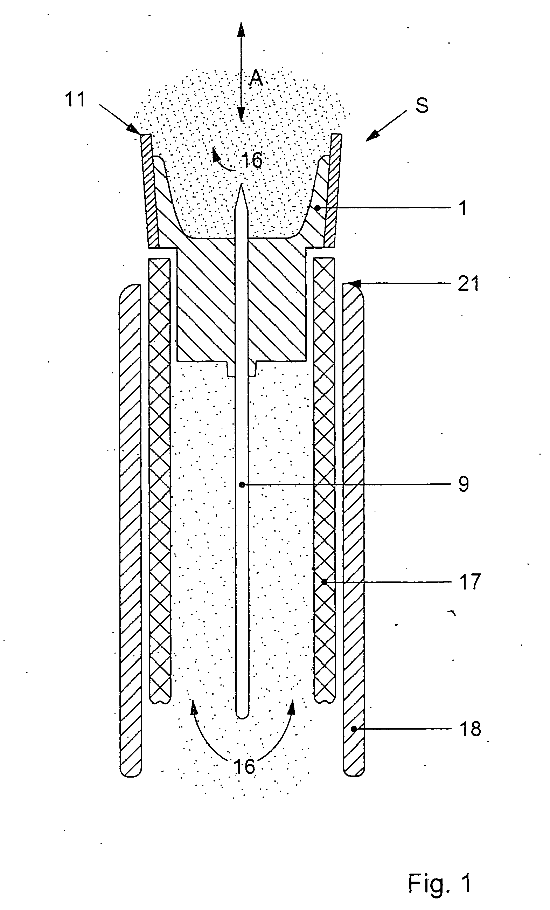

In Figur 1 ist das distale Ende eines Herzkatheters im Schnitt dargestellt. Der Herzkatheter umfasst einen Führungskatheter 18, in dem eine Sonde S in axialer Richtung (Pfeil A) verschiebbar angeordnet ist. Die Behandlung pathologischer Areale erfolgt dadurch, dass die Sonde S senkrecht auf den zu behandelnden Bereich aufgesetzt und das Gewebe mittels Laserlicht bestrahlt wird. Dabei wird die Sonde S über einen Kunststoffschlauch 17 betätigt, der an einem proximalen Abschnitt 2 der Sonde S befestigt ist.

Zur Kühlung des Endokards (Herzinnenhaut) und zur Vermeidung des Eindringens von Blut aus der Umgebung in den Behandlungsraum wird physiologische Kochsalzlösung 16 durch den Schlauch 17 und Durchlässe 15 im Sondenkörper 1 in den Behandlungsraum 13 geleitet.

Damit die Spülflüssigkeit aus dem Hohlraum austreten kann, ist der distale Rand 20 des Sondenkörpers 1 mit Kerben 14 versehen.

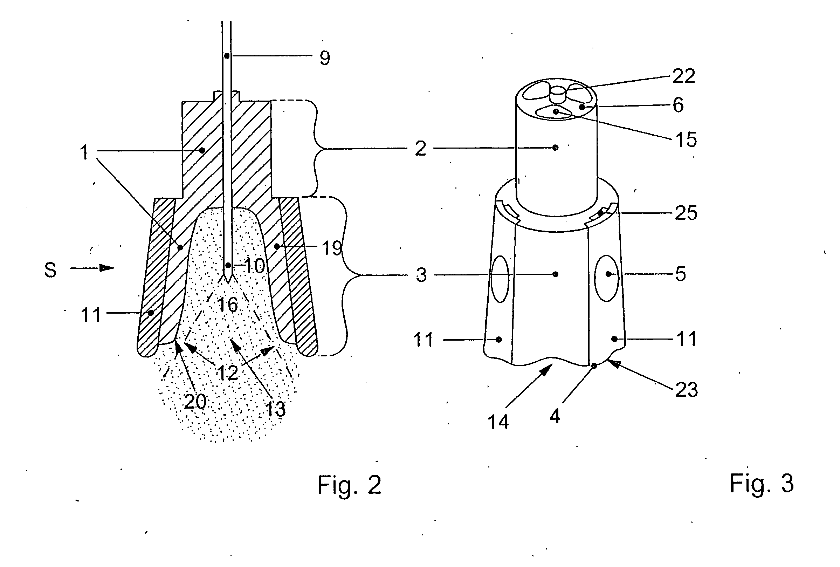

Der Aufbau einer beispielhaften Sonde S ist in Figur 2 genauer dargestellt. Die Sonde S besteht aus einem einstückigen Sondenkörper 1 (vorzugsweise aus Kunststoff) mit einem proximalen Abschnitt 2 und einem distalen Abschnitt 3, wobei der proximale Abschnitt 2 einen kleineren Querschnitt aufweist als der proximale Abschnitt 3. Der Sondenkörper 1 hat eine zentrale Öffnung 8 für eine optische Faser 9, deren Spitze 10 in den Hohlraum 13 hinein ragt.

Der Hohlraum 13 bzw. Behandlungsraum ist im distalen Abschnitt 3 des Sondenkörpers 1 gebildet und von einer im wesentlichen starren Wand 19 begrenzt. Der Sondenkörper 1 hat am distalen Ende eine Öffnung oder ist zumindest zum Gewebe hin lichtdurchlässig gebildet. Die den Hohlraum umgebende Wand des distalen Abschnitts ist dabei so stabil gebildet, dass sie dem äußeren Blutdruck ohne weiteres standhält und in der Lage ist, das Blut aus dem Raum um das distale Faserende fernzuhalten, damit sich das ausgestrahlte Licht ungehindert im Hohlraum ausbreiten kann.

Zur Behandlung pathologischer Areale ist die Spitze 10 der optischen Faser 9 im Sondenkörper 1 befestigt und wird somit beim Aufsetzen der Sonde immer in einem definierten Abstand zum Gewebe gehalten.

Der Querschnitt des Hohlraums 13 ist wesentlich größer und insbesondere wenigstens doppelt so groß wie der Querschnitt der Öffnung 8 für die optische Faser 9. Außerdem ist die axiale Erstreckung der Wand 19 derart dimensioniert, dass sich das von der Spitze 10 der optischen Faser 9 ausgestrahlte Licht - der Lichtkegel ist mit 12 bezeichnet - ungehindert ausbreiten kann, ohne den distalen Rand 20 des Sondenkörpers 1 zu treffen.

Der Sondenkörper 1 umfasst ferner drei plättchenförmige Elektroden 11, die in Ausnehmungen 4 am Außenumfang des distalen Abschnitts 3 angeordnet sind. Die Elektroden 11 schließen außen bündig zur Oberfläche des distalen Abschnitts 3 ab. Dadurch ist ein ungehindertes Durchschieben der Sonde durch Einführschleusen, Führungskatheter oder hämostatische Ventile gewährleistet.

Der am proximalen Abschnitt 2 befestigte Schlauch 17 schließt vorzugsweise ebenfalls bündig mit der Außenfläche des distalen Abschnitts 3 ab.

Wie in Figur 2 zu erkennen ist, ragen die Elektroden 11 nur geringfügig über den distalen Rand 20 des proximalen Abschnitts 3 hervor. Dabei sind sie insbesondere derart angeordnet, dass sie vom Laserlicht nicht bestrahlt werden. Eine zu starke Erhitzung der Elektroden 11 ist somit ausgeschlossen.

Figur 3 zeigt eine seitliche Ansicht des Sondenkörpers 1 von Figur 2, bei der die Ausnehmungen 4 und Befestigungspunkte 5 für die Elektroden 11 gut zu erkennen sind. Die Befestigungspunkte 5 (Vorsprünge) dienen zur Befestigung der Elektroden 11, die entsprechende Öffnungen aufweisen. Durch Wärmebehandlung werden die Elektroden 11 schließlich fixiert.

Zum Kontaktieren der Elektroden 11 mittels eines Drahtes ist eine zusätzliche Vertiefung 25 vorgesehen.

Der proximale Abschnitt 2 umfasst mehrere Durchlässe 15 zur Zuleitung von physiologischer Kochsalzlösung in den Hohlraum 13, die durch Stegwände 6 unterteilt sind. Am proximalen Ende des Sondenkörpers 1 (oben) ist ferner ein Ansatz 22 zur Aufnahme der optischen Faser 9 gebildet, der geringfügig über den proximalen Abschnitt 2 vorragt.

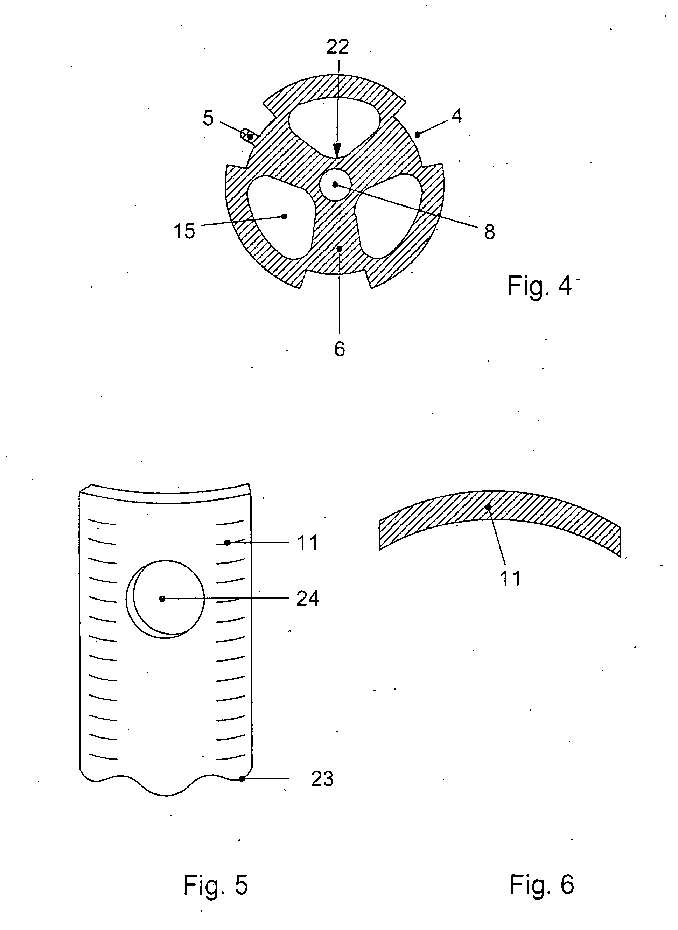

Figur 4 zeigt eine Aufsicht auf den Sondenkörper 1 von Figur 2. Wie zu erkennen ist, sind drei Durchlässe 15 für die Zuleitung von physiologischer Kochsalzlösung vorgesehen, die durch drei Stegwände 6 unterteilt sind. Die Ansatz 22 mit der Aufnahme 8 erstreckt sich in der Mitte des Sondenkörpers 1.

Figur 5 zeigt eine seitliche Ansicht einer plättchenförmigen Elektrode 11 mit einer kreisförmigen Öffnung 24, die auf einen Befestigungspunkt 5 aufgesetzt wird. Die Plättchenelektrode 11 hat einen geriffelten distalen Rand 23, der ein Verrutschen der Sonde auf dem Gewebe verhindert.

In Figur 6 ist eine Aufsicht auf die Elektrode von Figur 5 dargestellt. Die Elektrode 11 ist im wesentlichen ringsegmentförmig gekrümmt und hat dadurch eine relativ breite Kontaktfläche. Wegen der nur wenig über den Rand 20 vorragenden Platzierung der Elektroden 11 und deren breitflächigem Kontakt mit der Herzwand wird die Verletzungsgefahr bei der Applikation stark reduziert.

### Bezugszeichenliste

- 1: Sondenkörper
- 2: Proximaler Abschnitt
- 3: Distaler Abschnitt
- 4: Ausnehmung
- 5: Befestigungspunkt
- 6: Stegwand
- 7: Distaler Rand
- 8: Öffnung
- 9: Optische Faser
- 10: Faserspitze
- 11: Elektrode
- 12: Ausbreitungsrichtung.
- 13: Hohlraum
- 14: Kerbe
- 15: Durchlässe
- 16: Physiologische Lösung
- 17: Flexibler Schlauch
- 18: Führungskatheter
- 19: Wand
- 20: Distaler Rand
- 21: Distales Ende
- 22: Ansatz
- 23: Geriffelter Rand
- 24: Öffnung
- 25: Ausnehmung für Anschlussdraht
- A: Axialrichtung
- S: Sonde

## Patentansprüche

1. Herzkatheter, insbesondere zur Behandlung pathologischer Areale an den Herzwänden, mit
- einem schlauchförmigen Führungskatheter (18),
- einer Sonde (S) zum Aufsetzen auf die pathologischen Areale,
- einem flexiblen Führungsschlauch (17) zum Bewegen der Sonde (S) in Axialrichtung des Führungskatheters (18), und
- einer optischen Faser (9) zum Bestrahlen der pathologischen Areale, wobei die optische Faser (9) eine Spitze (10) aufweist, die das Licht kegelförmig ausstrahlt,
wobei :
- die Sonde (S) einen Sondenkörper (1) mit einer Aufnahme (8) für eine optische Faser (9) umfasst, in dem die spitze (10) der optischen Faser (9) in einem bestimmten Abstand zum Gewebe befestigt ist,
- der Sondenkörper (1) einen distalen Abschnitt (3) mit einem Hohlraum (13) aufweist, der umfangsseitig von einer im wesentlichen starren Wand (19) begrenzt ist, die in der Lage ist, das Blut aus dem Hohlraum (13) fernzuhalten, und der einen Behandlungsraum bildet, in dem sich das von der optischen Faser (9) ausgestrahlte Licht bis auf das zu behandelnde Gewebe ausbreiten kann, ohne den distalen Rand der Wand (19) zu treffen,
- der Führungsschlauch (17) an einem proximalen Abschnitt des Sondenkörpers (1) befestigt ist, und
- der Sondenkörper (1) wenigstens einen Durchlass zum Zuführen von Spülflüssigkeit in den Hohlraum (13) aufweist.

2. Herzkatheter nach Anspruch 1,
**dadurch gekennzeichnet,**
**dass** der Hohlraum (13) einen größeren Querschnitt aufweist als die Aufnahme (8) für die Faser (9).

3. Herzkatheter nach Anspruch 1 oder 2,
**dadurch gekennzeichnet,**
**dass** der Hohlraum (13) so dimensioniert ist, dass sich das von der Faser (9) ausgestrahlte Licht kegelförmig auf das Gewebe ausbreiten kann, wenn eine Spitze (10) der Faser (9) in Abstand zum Gewebe angeordnet ist.

4. Herzkatheter nach Anspruch 1,2 oder 3
**dadurch gekennzeichnet,**
**dass** der Sondenkörpers (1) einen proximalen Abschnitt (2) mit einem kleinen Querschnitt und einen distalen Abschnitt (3) mit einem größeren Querschnitt aufweist.

5. Herzkatheter nach einem der vorhergehenden Ansprüche,
**dadurch gekennzeichnet,**
**dass** der Sondenkörper (1) einstückig gebildet ist.

6. Herzkatheter nach einem der vorhergehenden Ansprüche,
**dadurch gekennzeichnet**,
die Sonde (S) eine oder mehrere Elektroden (11) zum Lokalsieren der zu behandelnden Areale aufweist.

7. Herzkatheter nach Anspruch 6,
**dadurch gekennzeichnet,**
**dass** die Elektroden (11) am Außenumfang des Sondenkörpers (1) befestigt sind.

8. Herzkatheter nach Anspruch 6 oder 7,
**dadurch gekennzeichnet,**
**dass** die Elektroden (11) in Ausnehmungen (4) des Sondekörpers (1) angebracht- sind, so dass sie bündig mit der Außenfläche des Sondenkörpers (1) abschließen.

9. Herzkatheter nach einem der Ansprüche 6 bis 8,
**dadurch gekennzeichnete**
**dass** die Elektroden (11) nicht oder nur geringfügig über den distalen Rand (20) des Sondenkörpers (1) vorragen.

10. Herzkatheter nach einem der Ansprüche 6 bis 9,
**dadurch gekennzeichnet,**
**dass** die Elektroden (11) plättchenförmig gebildet sind.

11. Herzkatheter nach einem der vorhergehenden Ansprüche,
**dadurch gekennzeichnet,**
**dass** der Sondenkörper (1) aus einem nicht elastischen Material besteht.

## Claims

1. Heart catheter, particularly for the treatment of pathological areas of the cardiac walls, comprising:
- A tubular guiding catheter (18),
- A probe (S) to be placed on the pathological areas,
- A flexible guiding tube (17) for moving the probe (S) in the axial direction of the guiding catheter (18), and
- An optical fiber (9) to irradiate the pathological areas, where the optical fiber (9) comprises a tip (10), which radiates the light conically, where:
- The probe (S) having a body (1) with a receptacle (8) for an optical fiber (9) wherein the tip (10) of the optical fiber (9) is mounted at a certain distance to the tissue,
- The probe body (1) comprising a distal portion (3) having a cavity (13) which is bounded circumferentially by a substantially rigid wall (19) that is able to keep the blood from the cavity (13) which thereby forms a treatment area in which the light emitted by the optical fiber (9) can spread onto the target tissue without streaking the distal edge of the wall (19),
- The guide tube (17) is fixed at a proximal portion of the probe body (1), and
- The probe body (1) comprises at least one passage for supplying irrigation fluid into the cavity (13).

2. Heart catheter according to claim 1, wherein said cavity (13) identifies a cross-sectional area larger than the receptacle (8) for the fiber (9).

3. Heart catheter according to claim 1 and 2, wherein the cavity (13) is sized such that a divergent light beam radiated by the optical fiber (9) can propagate towards the tissue, if the tip (10) of the fiber (9) is positioned at a given distance from the tissue.

4. Heart catheter according to claim 1,2 or 3, wherein the probe body (1) comprises a proximal portion (2) with a small cross-section, and a distal portion (3) with a bigger cross-section.

5. Heart catheter according to claim 1, 2, 3 or 4, wherein the probe body (1) is made of a single piece.

6. Heart catheter according to claim 1, wherein the probe (S) comprises one or several electrodes (11) for localization of target areas.

7. Heart catheter according to claim 6, wherein the electrodes (11) are mounted on the outer circumference of the probe body (1).

8. Heart catheter according to claim 6 or 7, wherein the electrodes (11) are mounted into recesses (4) of the probe body (1) such that they are flush with the outer circumference of the probe body (1).

9. Heart catheter according to one of claims 6 to 8, wherein the electrodes (11) do not or do only slightly protrude beyond a distal edge (20) of the probe body (1).

10. Heart catheter according to one of claims 6 to 9, wherein the electrodes (11) have a plate-like shape.

11. Heart catheter according to claim 1, wherein the probe body is made of a non-flexible material.

## Revendications

1. Cathéter cardiaque, en particulier pour le traitement des zones pathologiques des parois cardiaques, avec:
- Un cathéter de guidage tubulaire (18),
- Une sonde (S) d'être mis sur les zones pathologiques,
- Un souple guidage tubulaire (17) pour déplacer la sonde (S) dans le sens axial du cathéter de guidage (18), et
- Une fibre optique (9) pour irradier les zones pathologiques, où la fibre optique (9) comprend un pointe (10), qui irradie le cône de lumière, où:
- La sonde (S) comportant un corps (1) avec un récipient (8) pour une fibre optique (9) dans lequel la pointe (10) de la fibre optique (9) est monté à une certaine distance du tissu,
- Le corps de sonde (1) comprenant une partie distale (3) ayant une cavité (13) qui est délimitée circonférentiellement par une paroi sensiblement rigide (19) qui est capable de garder le sang de la cavité (13) qui forme ainsi un traitement domaine dans lequel le cône de lumière émise par la fibre optique (9) peut se répandre sur le tissu cible sans toucher le bord distal de la paroi (19),
- Le tube de guidage (17) est fixé à une portion proximale du corps de sonde (1), et
- Le corps de sonde (1) comprend au moins un passage d'alimentation en fluide d'irrigation dans la cavité (13).

2. Cathéter cardiaque selon la revendication 1, dans lequel ladite cavité (13) identifie une zone transversale plus grande que le réceptacle (8) pour la fibre (9).

3. Cathéter cardiaque selon la revendication 1 et 2, dans lequel la cavité (13) est dimensionné de telle sorte que un faisceau lumineux divergent rayonnée par la fibre optique (9) peut se propager vers le tissu, si la pointe (10) de la fibre (9) est positionné à une distance donnée à partir des tissus.

4. Cathéter cardiaque selon la revendication 1, 2 ou 3, dans lequel le corps de sonde (1) comporte une portion proximale (2) avec une petite section, et une partie distale (3) avec une plus grande section transversale.

5. Cathéter cardiaque selon la revendication 1, 2, 3 ou 4, dans lequel le corps de sonde (1) est faite d'une seule pièce.

6. Cathéter cardiaque selon la revendication 1, dans lequel la sonde (S) comprend une ou plusieurs électrodes (11) pour la localisation des zones cibles.

7. Cathéter cardiaque selon la revendication 6, dans lequel les électrodes (11) sont montés sur la circonférence extérieure du corps de sonde (1).

8. Cathéter cardiaque selon la revendication 6 ou 7, dans lequel les électrodes (11) sont montés dans des évidements (4) du corps de sonde (1) tels qu'ils sont à égalité avec la circonférence extérieure du corps de sonde (1).

9. Cathéter cardiaque selon l'une des revendications 6 à 8, dans lequel les électrodes (11) ne font pas ou ne faire que peu saillie au-delà un bord distal (20) du corps de sonde (1).

10. Cathéter cardiaque selon l'une des revendications 6 à 9, dans lequel les électrodes (11) ont une forme analogue à une plaque.

11. Cathéter cardiaque selon la revendication 1, dans lequel le corps de sonde est faite d'un matériau non flexibles.
